(19) Europäisches Patentamt
European Patent Office
Office européen des brevets

(11) Veröffentlichungsnummer: **0 000 126**
**B1**

(12) # EUROPÄISCHE PATENTSCHRIFT

(45) Veröffentlichungstag der Patentschrift: **01.12.82**

(51) Int. Cl.[3]: **C 07 H 15/04,** C 07 H 15/18, A 61 K 31/70 // C07H13/12

(21) Anmeldenummer: **78100115.1**

(22) Anmeldetag: **07.06.78**

(54) **Glucofuranosid-6-yl-Nitrosoharnstoffderivate, Verfahren zu ihrer Herstellung und sie enthaltende Arzneimittel.**

(30) Priorität: **15.06.77 CH 7359/77**

(43) Veröffentlichungstag der Anmeldung: **10.01.79 Patentblatt 79/1**

(45) Bekanntmachung des Hinweises auf die Patenterteilung: **01.12.82 Patentblatt 82/48**

(84) Benannte Vertragsstaaten: **BE CH DE FR GB NL SE**

(56) Entgegenhaltungen:
**CHEMICAL ABSTRACTS 85 (1976) 94652u & JA-A-76 26819 (TOKYO TANABE CO)**
**CHEMICAL ABSTRACTS 86 (1977) 16911j & JA-A-76 75 072 (TOKYO TANABE CO)**

(73) Patentinhaber: **CIBA-GEIGY AG, Patentabteilung Postfach, CH-4002 Basel (CH)**

(72) Erfinder: **Stanek, Jaroslav, Dr., Florastrasse 6, CH-4127 Birsfelden (CH)**

**EP 0 000 126 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Glucofuranosid-6-yl-Nitrosoharnstoffderivate, Verfahren zu ihrer Herstellung und sie enthaltende Arzneimittel

Die Erfindung betrifft neue $N_1$-Glucofuranosid-6-yl-$N_2$-nitrosoharnstoffe der Formel

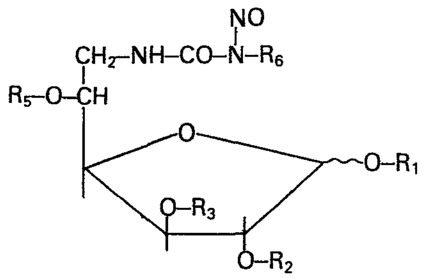
(I)

worin $R_1$ und $R_2$ Wasserstoff, gegebenenfalls durch freie oder verätherte Hydroxygruppen oder Halogen substituiertes Alkyl oder gegebenenfalls durch Niederalkyl, freies oder veräthertes Hydroxy, Halogen oder Trifluormethyl substituiertes Aralkyl oder Acyl, $R_1$ und $R_2$ zusammen auch Alkyliden oder Cycloalkyliden darstellen, $R_3$ und $R_5$ gegebenenfalls durch freie oder verätherte Hydroxygruppen oder Halogen substituiertes Alkyl oder gegebenenfalls durch Niederalkyl, freies oder veräthertes Hydroxy, Halogen oder Trifluoromethyl substituiertes Aralkyl oder Acyl, $R_3$ und $R_5$ zusammen auch Alkyliden oder Cycloalkyliden bedeuten, und $R_6$ gegebenenfalls durch freie oder verätherte Hydroxygruppen oder Halogen substituiertes Alkyl bedeutet.

Nachfolgend mit dem Ausdruck «nieder» modifizierte Reste, Radikale oder Verbindungen enthalten, sofern nichts besonderes angegeben, bis zu 7, in erster Linie bis zu 4 Kohlenstoffatomen.

Alkyl ist insbesondere Niederalkyl, z. B. Isopropyl, geradkettiges oder verzweigtes, in beliebiger Stellung gebundenes Butyl, Pentyl, Hexyl oder Heptyl und vor allem Methyl, Äthyl oder n-Propyl.

Als Substituenten der gegebenenfalls substituierten Alkylgruppe kommen in erster Linie in Betracht freie oder verätherte Hydroxygruppen, z. B. Niederalkoxygruppen, oder Halogenatome. Dabei kann der substituierte Alkylrest, wie Niederalkylrest, einen, zwei oder mehrere gleiche oder verschiedene Substituenten, insbesondere freie Hydroxylgruppen oder Halogenatome tragen.

Aralkyl ist insbesondere Arylniederalkyl, wobei der Niederalkylteil vor allem dem obgenannten Niederalkyl entspricht, und in erster Linie Methyl oder Äthyl ist. Der aromatische Teil ist insbesondere ein mono-cyclischer, sowie bi-cyclischer Arylrest, in erster Linie Phenyl, aber auch Naphthyl. Er kann gegebenenfalls, z. B. durch Niederalkylgruppen, freies oder veräthertes Hydroxy, z. B. Niederalkoxy oder Niederalkylendioxy, Halogenatome und/oder Trifluormethyl mono-, di- oder polysubstituiert sein. Besonders zu nennen sind 2-Phenyläthyl, Chlorbenzyl, Methylbenzyl, Hydroxybenzyl, Methoxybenzyl oder vor allem Benzyl.

Der Alkylidenrest ist insbesondere ein Niederalkylidenrest, wie der 2-Butyliden-, 3-Pentyliden- und in erster Linie Isopropylidenrest.

Der Cycloalkylidenrest enthält vorzugsweise 5–7 Ringkohlenstoffatome und ist in erster Linie Cyclopentyliden oder Cyclohexyliden.

Acyl ist insbesondere ein Acylrest einer organischen Säure, insbesondere einer organischen Carbonsäure. So ist Acyl insbesondere Alkanoyl, vor allem mit 2–18 Kohlenstoffatomen, wie Acetyl oder Propionyl, oder auch Aroyl, wie Naphthoyl-1, Naphthoyl-2- und insbesondere Benzoyl oder durch Halogen, Niederalkyl, Niederalkoxy, Trifluormethyl, Hydroxy oder Niederalkanoyloxy substituiertes Benzoyl oder Naphthoyl. Acyl kann auch ein Acylrest einer organischen Sulfonsäure sein, z. B. einer Alkansulfonsäure, insbesondere einer Niederalkansulfonsäure, wie Methansulfonsäure oder Äthansulfonsäure, oder einer Arylsulfonsäure, insbesondere einer gegebenenfalls niederalkyl-substituierten Phenylsulfonsäure, wie Benzolsulfonsäure oder p-Toluolsulfonsäure, sowie den Rest einer Carbaminsäure darstellen, wie unsubstituiertes Carbamoyl, Niederalkyl-carbamoyl oder Aryl-carbamoyl, wie Methylcarbamoyl oder Phenyl-carbamoyl.

Niederalkyl, als Substituent der obgenannten Reste ist in erster Linie Methyl oder Äthyl, aber auch n-Propyl, Isopropyl oder geradkettiges oder verzweigtes Butyl.

Niederalkoxy, als Substituent der obgenannten Reste, ist insbesondere Methoxy oder Äthoxy, ferner n-Propoxy, Isopropoxy, n-Butoxy oder Isobutoxy.

Halogen ist z. B. Fluor, Chlor oder Brom.

Die neuen Verbindungen können in der Form von Anomerengemischen oder von reinen α- oder β-Anomeren vorliegen.

Die neuen Verbindungen besitzen wertvolle pharmakologische Eigenschaften, insbesondere zeigen sie eine sehr gute Wirkung bei einigen verschiedenartigen transplantablen Tumoren und Leukämien, wie auch zum Teil bei Virus-induzierter Leukämie. So bewirken sie in Dosen von 25–500 mg/kg i.p. eine starke Hemmung des Tumorwachstums bei Mäusen mit z. B. Ehrlich-Ascites-Karzinom oder solidem Harding-Passey-Melanom, und bei Ratten mit z. B. Yoshida-Ascites-Sarcom. Analoge Dosen bewirken eine Lebensverlängerung gegenüber Kontrollen bei Mäusen mit z. B. Leukämie L 1210 oder Rauscher-Leukämie.

So erreicht man z. B. mit dem Äthyl-6-desoxy-3,5-di-O-methyl-6-(3-methyl-3-nitroso-ureido)-β-D-glucofuranosid in Dosen von 50–250 mg/kg i.p. eine 800–100%ige Hemmung des Wachstums der genannten Tumoren und bei Leukämie L 1210 eine Lebensverlängerung von ca. 60%, sowie nach peroraler Applikation bei Mäusen mit Rauscher-Leukämie eine Lebensverlängerung von ca. 150%. Die Verträglichkeit ist gut. Nebenwirkungen sind auch bei längerer Behandlung nicht festzustellen. Auch bei normalen Tieren sind nach dreiwöchiger peroraler Behandlung makroskopisch keine Organveränderungen zu sehen.

Zuckerderivate, welche die Nitrosoharnstoffgruppierung enthalten, sind aus der Literatur bekannt. So werden in Chemical Abstracts 85, 94652u (1976) Alkyl-N-carbamyl-N-2-chloräthyl-N'-nitroso-D-glucopyranoside und in Chemical Abstracts 86, 16911j (1977) 5-Nitrosoureido-5-deoxy-D-ribofuranosederivate als tumorhemmende Wirkstoffe vorgeschlagen.

Diesen gegenüber zeigen die erfindungsgemässen Verbindungen ein breites Wirkungsspektrum nicht nur gegen transplantable Ascitestumoren (CrSa 180, Ehrlich Karzinom), sondern vor allem auch gegen verschiedene solide, transplantable oder auf chemischem Wege induzierte Tumoren.

Die Erfindung betrifft insbesondere Verbindungen der Formel I, worin $R_1$ und $R_2$ Wasserstoff, gegebenenfalls durch Hydroxy, Niederalkoxy oder Halogen substituiertes Niederalkyl, gegebenenfalls durch Hydroxy, Niederalkoxy, Halogen oder Trifluormethyl, in erster Linie in para-Stellung, substituiertes Benzyl, $R_1$ und $R_2$ auch Niederalkyliden oder Cycloalkyliden mit 5–6 Kohlenstoffatomen bedeutet, $R_3$ und $R_5$ gegebenenfalls durch Hydroxy, Niederalkoxy oder Halogen substituiertes Niederalkyl, gegebenenfalls durch Hydroxy, Niederalkoxy, Halogen oder Trifluormethyl, in erster Linie in p-Stellung, substituiertes Benzyl, oder Niederalkanoyl, z. B. Acetyl oder Propionyl, oder gegebenenfalls durch Halogen, Niederalkoxy, Hydroxy oder Niederalkanoyloxy substituiertes Benzoyl, z. B. p-Chlorbenzoyl, p-Brombenzoyl, p-Methoxybenzoyl oder o- oder p-Hydroxybenzoyl, zusammen jedoch auch Niederalkyliden oder Cycloalkyliden mit 5–6 Kohlenstoffatomen bedeuten und $R_6$ gegebenenfalls durch Halogen, Hydroxy oder Niederalkoxy substituiertes Niederalkyl bedeutet.

Besonders wertvoll sind dabei Verbindungen, in denen $R_3$ und $R_5$ einen gleichen Rest darstellen.

Hervorzuheben sind insbesondere Verbindungen obiger Formel I, worin $R_1$ Niederalkyl und $R_2$ Wasserstoff oder $R_1$ und $R_2$ zusammen Niederalkyliden bedeuten, $R_3$ und $R_5$ Niederalkyl oder gegebenenfalls durch Halogen, Hydroxy, Niederalkoxy oder Alkyl, besonders in p-Stellung, substituiertes Benzyl oder $R_6$ gegebenenfalls mit Chlor substituiertes Niederalkyl, z. B. Methyl oder Chloräthyl darstellen.

In erster Linie sind Verbindungen zu nennen, worin $R_3$ und $R_5$ Methyl, $R_6$ Methyl oder Chloräthyl und $R_1$ Wasserstoff, Methyl, Äthyl oder Propyl und $R_2$ Wasserstoff oder $R_1$ und $R_2$ zusammen den Isopropylidenrest bedeuten.

Die neuen Verbindungen werden erhalten, wenn man eine Verbindung der Formel II

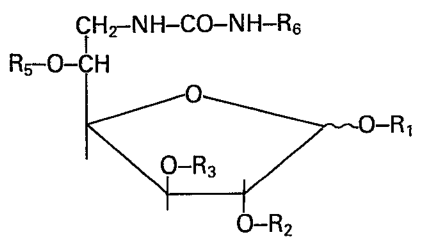

(II);

in an sich bekannter Weise nitrosiert.

Dazu setzt man vorzugsweise die Verbindung II mit salpetriger Säure, deren Salzen oder Derivaten um. Vorzugsweise verwendet man dazu ein Salz, wie ein Alkali- oder Erdalkali-, besonders das Natriumsalz der salpetrigen Säure und setzt daraus mit einer Säure, wie einer Mineralsäure, z. B. Salzsäure, Schwefelsäure oder Salpetersäure, einer organischen Säure, wie Kohlensäure, Essigsäure oder einer Sulfonsäure, z. B. einer Niederalkansulfonsäure, wie Methan- oder Äthansulfonsäure oder einem Sulfonsäuregruppen enthaltenden Ionenaustauscher, z. B. Amberlite IR 120, die salpetrige Säure frei. Man kann aber auch ein Anhydrid der salpetrigen Säure, insbesondere ein gemischtes Anhydrid mit z. B. der Salpetersäure oder einer Halogenwasserstoffsäure, verwenden.

Wenn notwendig, arbeitet man in Gegenwart eines Lösungsmittels, wobei z. B. eine vorhandene organische Säure ebenfalls als solches verwendet werden kann. Die Reaktion wird vorzugsweise bei tiefer Temperatur, z. B. −10°C bis 30°C durchgeführt.

Die bei dieser Verfahrensmethode verwendeten Ausgangsstoffe sind neu. Sie lassen sich in an sich bekannter Weise aus einer entsprechenden, in 6-Stellung unsubstituierten Glucofuranose gewinnen, z. B. durch Umsetzen zu einem reaktionsfähigen Ester, z. B. mit einer Alkansulfonsäure, Arylsulfonsäure oder Halogenwasserstoffsäure, dann zu einem Azid und Reduktion des so erhaltenen Azids zur 6-Desoxy-6-amino-glucofuranose, welche dann mit einem geeigneten N-$R_6$-Carbaminsäurederivat, wie einem entsprechenden Isocyanat zur 6-Desoxy-6-(-3-$R_6$-ureido)-glucofuranose kondensiert wird. Wie oben erwähnt erfolgen diese Umsetzungen in an sich bekannter Weise.

Eine weitere Methode zur Herstellung der neuen Nitrosoharnstoffe besteht darin, dass man eine Verbindung der Formel III

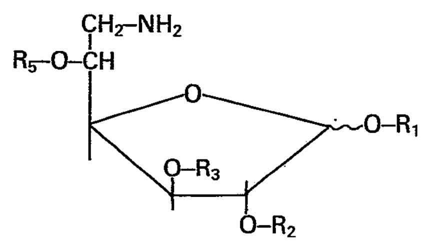

(III)

mit einem reaktionsfähigen Derivat einer

N-Nitroso-N-$R_6$-carbaminsäure (IV)

umsetzt.

Das reaktionsfähige Derivat kann beispielsweise ein Säureanhydrid, vorzugsweise ein gemischtes Säureanhydrid, wie ein Säureazid oder ein aktivierter Ester sein. Als aktivierte Ester seien insbesondere genannt Cyanmethylester, Carboxymethylester, Paranitrophenylthioester, Paranitrophenylester, 2,4,5-Trichlorphenylester, Pentachlorphenylester, N-Hydroxy-succinimidester, N-Hydroxy-phalimidester, 8-Hydroxychinolinester oder N-Hydroxypiperidinester.

Diese Reaktion führt man in an sich bekannter Weise durch, vorzugsweise in einem Lösungsmittel, wie Wasser, einem halogenierten Kohlenwasserstoff, z. B. Dichlor- oder Trichloräthan, einem Äther, wie Diäthyläther, Tetrahydrofuran, Dimethylformamid, Dimethylsufoxyd, oder einem gegebenenfalls alkylierten Pyridin, wie Pyridin, Picolin, Lutidin, oder Chinolin.

Die verwendeten Ausgangsstoffe sind bekannt oder lassen sich in an sich bekannter Weise herstellen. So kann man das Amin der Formel III aus einer entsprechenden, in 6-Stellung unsubstituierten Glucofuranose, z. B. durch Umsetzen zu einem reaktionsfähigen Ester, z. B. mit einer Alkan- oder Arylsulfonsäure oder einer Halogenwasserstoffsäure, und dann zu einem Azid und Reduktion des so erhaltenen Azid zu 6-Desoxy-6-aminoglucofuranose gewinnen.

Die oben beschriebenen Verfahren werden nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer inerten Atmosphäre, z. B. unter Stickstoff.

Die neuen Verbindungen können als reine α- oder β-Anomere oder als Anomerengemische vorliegen. Letztere können aufgrund der physikalischchemischen Unterschiede oder Bestandteile in bekannter Weise in die beiden reinen Anomeren aufgetrennt werden, z. B. mittels chromatographischer Trennung, wie Dünnschichtchromatographie oder irgendeines anderen geeigneten Trennverfahrens. Vorzugsweise isoliert man das wirksamere der beiden Anomeren.

Die Erfindung betrifft auch diejenigen Ausführungsformen des Verfahrens, bei denen man einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche Verbindungen der Formel I enthalten. Bei den erfindungsgemässen pharmazeutischen Präparaten handelt es sich um solche zur enteralen, wie oralen und rektalen sowie parenteralen Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüterspezies, dem Alter und dem individuellen Zustand sowie von der Applikationsweise ab.

Die neuen pharmazeutischen Präparate enthalten von etwa 10% bis etwa 95%, vorzugsweise von etwa 20% bis etwa 90% des Wirkstoffes. Erfindungsgemässe pharmazeutische Präparate können z. B. in Dosiseinheitsformen wie Dragées, Tabletten, Kapseln, Suppositorien oder Ampullen vorliegen. Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren hergestellt.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u. a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifizierung oder Kennzeichnung verschiedener Wirkstoffdosen, beigefügt werden.

Bei der Behandlung von mit Tumoren oder Leukämie behafteten Warmblütern zur Erzielung tumor- und/oder leukämiehemmender Wirkungen durch Verabfolgung eines erfindungsgemässen pharmazeutischen Präparates beträgt die Tagesdosis bei einem etwa 70 kg schweren Warmblüter etwa 10–500 mg pro Tag, vorzugsweise 50–300 mg pro Tag.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1

Eine auf 0°C gekühlte Lösung von 60,7 g Äthyl-3,5-di-O-methyl-6-deoxy-6-(3-methylureido)-α-D-glucofuranosid in 500 ml Wasser und 15 ml Eisessig wird innerhalb 15 min tropfenweise mit einer Lösung von 15,8 g Natriumnitrit in 80 ml Wasser versetzt, eine Stunde bei der gleichen Temperatur gerührt und 16 h bei Raumtemperatur stehen gelassen. Das auskristallisierte Produkt wird abgesaugt, mit wenig Eiswasser gewaschen und getrocknet. Die Mutterlauge extrahiert man mit Chloroform, trocknet die organische Phase über Magnesiumsulfat und destilliert das Lösungsmittel ab. Der Rückstand wird säulenchromatographisch auf Kieselgel mit Methylenchlorid/Essigester (85:15) gereinigt, das kristalline Produkt mit dem ersten Kristallisat vereinigt und aus Äther/Petroläther umkristallisiert. Das Äthyl-3,5-di-O-methyl-6-deoxy-6-(3-methyl-3-nitrosoureido)-α-D-gluco-

furanosid schmilzt bei 90°C; $R_f$-Wert 0,45 auf Kieselgeldünnschichtplatten im System Methylenchlorid/Methanol (15:1); $[\alpha]_D^{20} = +43° \pm 1°$ (Chloroform, c = 1,465).

Das Ausgangsmaterial kann wie folgt hergestellt werden:

Eine Lösung von 207 g 3,5-Di-O-methyl-1,2-O-isopropyliden-α-D-glucofuranose in 600 ml absolutem Pyridin wird unter Rühren und Aussenkühlung tropfenweise innerhalb von 45 min mit 67 ml Methansulfonsäurechlorid versetzt und 4 h bei Raumtemperatur stehen gelassen. Man gibt nun 50 ml Wasser zu und dampft nach weiteren 15 min die Hauptmenge Pyridin ab. Der Rückstand wird in Äther aufgenommen, die ätherische Lösung mit Wasser, eiskalter 2-n-Salzsäure, Wasser, einer gesättigten Natriumhydrogencarbonatlösung und Wasser gewaschen, über Magnesiumsulfat getrocknet und das Lösungsmittel abdestilliert. Der ölige Rückstand stellt die 3,5-Di-O-methyl-1,2-O-isopropyliden-6-O-mesyl-α-D-glucofuranose vom $R_f$-Wert 0,35 auf Kieselgeldünnschichtplatten im System Methylenchlorid/Essigester (85:15) dar.

240 g dieses Produktes werden in 1700 ml N,N-Dimethylformamid gelöst, mit 142 g Natriumazid und 170 ml Wasser versetzt und 3 h bei 110°C gerührt. Man kühlt das Reaktionsgemisch, filtriert und dampft das Filtrat ein. Der Rückstand wird in Äther aufgenommen, mit Wasser gewaschen, über Magnesiumsulfat getrocknet und das Lösungsmittel abdestilliert. Man erhält so die 6-Azido-6-deoxy-3,5-di-O-methyl-1,2-O-isopropyliden-α-D-glucofuranose als gelbliches Öl vom $R_f$-Wert 0,61 auf Kieselgeldünnschichtplatten im System Methylenchlorid/Essigester (85:15) und der optischen Drehung $[\alpha]_D^{20} = -57° \pm 1°$ (Chloroform, c = 1,915).

Eine Lösung von 193 g dieser Verbindung in 3500 ml 1-n-alkoholischer Salzsäure lässt man 18 h bei Raumtemperatur stehen. Anschliessend dampft man die Hauptmenge des Lösungsmittels im Wasserstrahlvakuum ab, nimmt den Rückstand in Äther auf und wäscht diese Lösung mit Wasser, einer gesättigten Natriumhydrogencarbonatlösung und Wasser, trocknet über Magnesiumsulfat und dampft zur Trockne ein. Das erhaltene Anomerengemisch wird säulenchromatographisch auf Kieselgel mit Methylenchlorid/Essigester (85:15) getrennt. Das Äthyl-6-azido-6-deoxy-3,5-di-O-methyl-α-D-glucofuranosid weist den $R_f$-Wert 0,32 auf Kieselgeldünnschichtplatten im System Methylenchlorid/Essigester (85:15) und die optische Drehung $[\alpha]_D^{20} = +56° \pm 1°$ (Chloroform, c = 0,89) auf. Das entsprechende β-Glucofuranosid zeigt im gleichen System den $R_f$-Wert 0,11.

21,9 g Äthyl-6-azido-6-deoxy-3,5-di-O-methyl-α-D-glucofuranosid in 200 ml Äthanol werden in Gegenwart von 2 g 5%-Palladium/Kohle mit Wasserstoff reduziert. Nach dem Abfiltrieren des Katalysators und Abdestillieren des Alkohols erhält man das Äthyl-6-amino-6-deoxy-3,5-di-O-methyl-α-D-glucofuranosid als gelbliches Öl.

Eine Lösung von 74,9 g dieses Öles in 550 ml Äthanol wird mit einer Lösung von 18,5 ml Methylisocyanat in 60 ml Methylenchlorid tropfenweise innerhalb einer Stunde versetzt und das Reaktionsgemisch zur Trockne eingedampft. Das erhaltene Äthyl-6-deoxy-3,5-di-O-methyl-6-(3-methyl-ureido)-α-D-glucofuranosid wird aus Essigester/Äther kristallisiert; Smp. 144°, $[\alpha]_D^{20} = +57° \pm 1°$ (Chloroform, c = 1,134) und $R_f$-Wert 0,22 auf Kieselgel im System Methylenchlorid/Methanol (15:1).

Beispiel 2

Eine auf 0–5 °C gekühlte Lösung von 33,4 g 3,5-D-O-methyl-6-deoxy-1,2-O-isopropyliden-6-(3-methylureido)-α-D-glucofuranose in 280 ml Wasser und 8,0 ml Eisessig wird innerhalb 30 min mit einer Lösung von 8,5 g Natriumnitrit in 40 ml Wasser tropfenweise versetzt. Man rührt eine Stunde bei 0,5° und 18 h bei Raumtemperatur. Anschliessend wird die Lösung mit Chloroform extrahiert, die organische Phase mit Wasser gewaschen, über Magnesiumsulfat getrocknet und zur Trockne eingedampft. Der Rückstand wird auf 1200 g Kieselgel mit Methylenchlorid/Essigester (85:15) chromatographiert. Die Fraktionen vom $R_f$-Wert 0,41 mit der 6-Deoxy-3,5-di-O-methyl-1,2-O-isopropyliden-6-(3-methyl-3-nitrosoureido)-α-D-glucofuranose werden zur Trockne eingedampft; $[\alpha]_D^{20} = -49° \pm 1°$ (Chloroform, c = 1,127).

Das verwendete Ausgangsmaterial kann wie folgt hergestellt werden:

60,0 g 6-Azido-6-deoxy-3,5-di-O-methyl-1,2-O-isopropyliden-α-D-glucofuranose werden in 600 ml Äthanol gelöst und in Gegenwart von 5%-Palladium/Kohle mit Wasserstoff reduziert. Nach dem Abtrennen des Katalysators und Abdestillieren des Lösungsmittels erhält man die 6-Amino-6-deoxy-3,5-di-O-methyl-1,2-O-isopropyliden-α-D-glucofuranose als farbloses Öl. 33,2 g dieses Produktes werden in 250 ml Äthanol gelöst und unter Rühren tropfenweise innerhalb 30 min mit einer Lösung von 8,4 ml Methylisocyanat in 25 ml Methylenchlorid versetzt. Nach weiteren 60 min Rühren wird das Reaktionsgemisch zur Trockne eingedampft und der Rückstand aus Aceton kristallisiert. Die so erhaltene 6-Deoxy-3,5-di-O-methyl-1,2-O-isopropyliden-6-(3-methylureido)-α-D-glucofuranose vom $R_f$-Wert 0,45 auf Kieselgel im System Aceton und $[\alpha]_D^{20} = -57° \pm 1°$ (Chloroform, c = 1,987) schmilzt bei 66–69°.

Beispiel 3

Eine auf 0–5°C gekühlte Lösung von 52,4 g Äthyl-6-deoxy-3,5-di-O-methyl-6-(3-methyl-ureido)-β-D-glucofuranosid in 420 ml Wasser und 24,5 ml Eisessig wird unter Rühren tropfenweise innerhalb 45 min mit einer Lösung von 25,4 g Natriumnitrit in 130 ml Wasser versetzt und 18 h bei der gleichen Innentemperatur gerührt. Anschliessend extrahiert man mit Chloroform, wäscht die organische Phase mit Wasser, trocknet über Mgnesiumsulfat und dampft zur Trockne ein. Der Rückstand wird auf Kieselgel mit Methylenchlorid/Essigester (85:15) chromatographiert. Die Fraktionen mit dem Äthyl-6-deoxy-3,5-di-O-methyl-6-(3-methyl-3-nitrosoureido)-β-D-glucofuranosid vom $R_f$-Wert 0,15 werden vereinigt und zur Trockne einge-

dampft: $[\alpha]^{20}_{D} = -57° \pm 1°$ (Chloroform, c = 1,858).

Das verwendete Ausgangsmaterial kann wie folgt hergestellt werden:

24 g Äthyl-6-azido-6-deoxy-3,5-di-O-methyl-β-D-glucofuranosid werden in 240 ml Methanol gelöst und in Gegenwart von 5%-Palladium/Kohle mit Wasserstoff reduziert. Der Katalysator wird abfiltriert und das Filtrat zur Trockne eingedampft. Das erhaltene 6-Amino-derivat wird direkt weiter umgesetzt.

Eine Lösung von 39,2 g davon in 330 ml Äthanol wird unter Rühren tropfenweise innerhalb von 40 min mit einer Lösung von 10 ml Methylisocyanat in 30 ml Methylenchlorid versetzt und 16 h bei Raumtemperatur stehen gelassen. Anschliessend wird das Reaktionsgemisch zur Trockne eingedampft und der Rückstand in Essigester aufgenommen. Man filtriert diese Lösung über Aktivkohle und dampft zur Trockne ein. Das erhaltene Öl stellt das Äthyl-6-deoxy-3,5-di-O-methyl-6-(3-methyl-ureido)-β-D-glucofuranosid vom $R_f$-Wert 0,10 auf Kieselgel im System Methylenchlorid/Methanol (15:1) dar.

Beispiel 4

In analoger Weise ausgehend von den entsprechenden Ausgangsstoffen werden die folgenden Verbindungen erhalten:

a) Äthyl-2-O-acetyl-6-deoxy-3,5-O-methyl-6-(3-methyl-3-nitrosoureido)-α-D-glucofuranosid, Öl $[\alpha]^{20}_{D} = +96° \pm 1°$ (Chloroform, c = 0,710)

b) Äthyl-6-(3-äthyl-3-nitrosoureido)-6-deoxy-3,5-di-O-methyl-α-D-glucofuranosid, Öl, $[\alpha]^{20}_{D} = +41° \pm 1°$ (Chloroform, c = 1,174)

c) Äthyl-6-[3-(2-chloräthyl)-3-nitrosoureido]-6-deoxy-3,5-di-O-methyl-D-glucofuranosid, Öl, $[\alpha]^{20}_{D} = +37° \pm 1°$ (Chloroform, c = 0,935)

d) Äthyl-6-(3-n-butyl-3-nitrosoureido)-6-deoxy-3,5-di-O-methyl-α-D-glucofuranosid, Öl $[\alpha]^{20}_{D} = +25° \pm 1°$ (Chloroform, c = 1,579)

e) Äthyl-6-deoxy-5-O-methyl-6-(3-methyl-3-nitrosoureido)-3-O-propyl-α-D-glucofuranosid, F. = 41°, $[\alpha]^{20}_{D} = +37° \pm 1°$ (Chloroform, c = 0,926)

f) Äthyl-5-O-äthyl-6-deoxy-6-(3-methyl-3-nitrosoureido)-3-O-propyl-α-D-glucofuranosid, F. = 60°, $[\alpha]^{20}_{D} = -28° \pm 1°$ (Chloroform, c = 1,362) und

g) Äthyl-3-O-benzyl-6-deoxy-5-O-methyl-6-(3-methyl-3-nitrosoureido)-α-D-glucofuranosid, F. = 92–93°, $[\alpha]^{20}_{D} = +32° \pm 1°$ (Chloroform, c = 1,438)

Beispiel 5

Eine auf 0°C gekühlte Lösung von 5,0 g Äthyl-6-amino-6-deoxy-3,5-di-O-methyl-α-D-glucofuranosid in 40 ml Chloroform wird unter Rühren mit einer Lösung von 2,5 g N-Nitroso-methylcarbamylazid in 40 ml Äther tropfenweise während 10 min versetzt und weiter 1 h im Eisbad und 3 h bei Raumtemperatur gerührt. Die Lösung wird nun auf die Hälfte eingeengt, mit eiskalter 2-n-Salzsäure, Wasser, einer gesättigten Natriumhydrogencarbonatlösung und Wasser gewaschen, über Magnesiumsulfat getrocknet und zur Trockne eingedampft. Der kristalline Rückstand von Äthyl-3,5-di-O-methyl-6-deoxy-6-(3-methyl-3-nitrosoureido)-α-D-glucofuranosid wird aus Äther/Petroläther umkristallisiert, Smp. 90°C, $[\alpha]^{20}_{D} = +43° \pm 1°$ (Chloroform, c = 1,102).

**Patentansprüche**

1. $N_1$-Glucofuranosid-6-yl-$N_3$-nitroso-harnstoffe der Formel

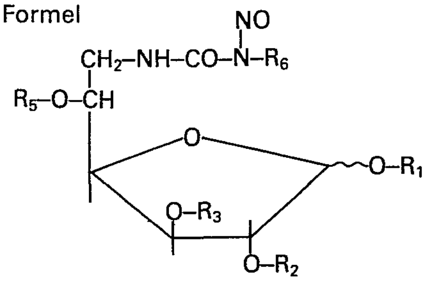

(I)

worin $R_1$ und $R_2$ Wasserstoff, gegebenenfalls durch freie oder verätherte Hydroxygruppen oder Halogen substituiertes Alkyl oder gegebenenfalls durch Niederalkyl, freies oder veräthertes Hydroxy, Halogen oder Trifluoromethyl substituiertes Aralkyl oder Acyl, $R_1$ und $R_2$ zusammen auch Alkyliden oder Cycloalkyliden darstellen, $R_3$ und $R_5$ gegebenenfalls durch freie oder verätherte Hydroxygruppen oder Halogen substituiertes Alkyl oder gegebenenfalls durch Niederalkyl, freies oder veräthertes Hydroxy, Halogen oder Trifluoromethyl substituiertes Aralkyl oder Acyl, $R_3$ und $R_5$ zusammen auch Alkyliden oder Cycloalkyliden bedeuten, und $R_6$ gegebenenfalls durch freie oder verätherte Hydroxygruppen oder Halogen substituiertes Alkyl bedeutet.

2. Verbindungen nach Anspruch 1, worin $R_1$ und $R_2$ Wasserstoff, gegebenenfalls durch Hydroxy, Niederalkoxy oder Halogen substituiertes Niederalkyl, gegebenenfalls durch Hydroxy, Niederalkoxy, Halogen oder Trifluormethyl substituiertes Benzyl, $R_1$ und $R_2$ auch Niederalkyliden oder Cycloalkyliden mit 5–6 Kohlenstoffatomen bedeuten, $R_3$ und $R_5$ gegebenenfalls durch Hydroxy, Niederalkoxy oder Halogen substituiertes Niederalkyl, gegebenenfalls durch Hydroxy, Niederalkoxy, Halogen oder Trifluormethyl substituiertes Benzyl, Niederalkanoyl oder gegebenenfalls durch Halogen, Niederalkoxy, Hydroxy oder Niederalkanoyloxy substituiertes Benzoyl, zusammen jedoch auch Niederalkyliden oder Cycloalkyliden mit 5–6 Kohlenstoffatomen bedeuten und $R_6$ ein gegebenenfalls durch Halogen, Hydroxy, oder Niederalkoxy substituierter Niederalkylrest ist.

3. Verbindungen nach Anspruch 2, worin der substituierte Benzylrest in para-Stellung substituiert ist.

4. Verbindungen nach Anspruch 1, worin $R_1$ Niederalkyl und $R_2$ Wasserstoff oder $R_1$ und $R_2$ zusammen Niederalkyliden bedeuten, $R_3$ und $R_5$ Niederalkyl oder gegebenenfalls durch Halogen, Hydroxy, Niederalkoxy oder Alkyl substituiertes Benzyl und $R_6$ gegebenenfalls mit Chlor substituiertes Niederalkyl darstellen.

5. Verbindungen nach Anspruch 1, worin $R_1$ Wasserstoff, Methyl, Äthyl oder Propyl und $R_2$ Wasserstoff und $R_1$ und $R_2$ zusammen den Isopropylidenrest bedeuten und $R_3$ und $R_5$ Methyl und $R_6$ Methyl oder Chloräthyl darstellen.

6. Die α- oder β-Anomeren der in den Patentansprüchen 1–5 genannten Verbindungen.

7. Äthyl-3,5-di-O-methyl-6-deoxy-6-(3-methyl-3-nitrosoureido-α-D-glucofuranosid oder -β-D-glucofuranosid.

8. 6-Deoxy-3,5-di-O-methyl-1,2-O-isopropyliden-6-(3-methyl-3-nitrosoureido)-α-D-glucofuranose.

9. Äthyl-2-O-acetyl-6-deoxy-3,5-di-O-methyl-6-(3-methyl-3-nitrosoureido)-D-glucofuranosid.

10. Äthyl-6-(3-äthyl-3-nitrosoureido)-6-deoxy-3,5-di-O-methyl-D-glucofuranosid.

11. Äthyl-6-[3-(2-chloräthyl)-3-nitrosoureido]-6-deoxy-3,5-di-O-methyl-D-glucofuranosid.

12. Äthyl-6-(3-n-butyl-3-nitrosoureido)-6-deoxy-3,5-di-O-methyl-D-glucofuranosid.

13. Äthyl-6-deoxy-5-O-methyl-6-(3-methyl-3-nitrosoureido)-3-O-propyl-D-glucofuranosid.

14. Äthyl-5-O-äthyl-6-deoxy-6-(3-methyl-3-nitrosoureido)-3-O-propyl-D-glucofuranosid.

15. Äthyl-3-O-benzyl-6-deoxy-5-O-methyl-6-(3-methyl-3-nitrosoureido)-D-glucofuranosid.

16. Pharmazeutische Präparate, enthaltend eine der in den Ansprüchen 1 bis 15 genannten Verbindungen.

17. Verbindungen nach Anspruch 1 zur Anwendung in einem Verfahren zur therapeutischen Behandlung des menschlichen oder tierischen Körpers.

18. Verbindungen nach Anspruch 1 als tumorhemmende Mittel.

19. Verwendung von Verbindungen nach Anspruch 1 zur Herstellung von pharmazeutischen Präparaten.

20. Verfahren zur Herstellung von $N_1$-Glucofuranosid-6-yl-$N_3$-nitroso-harnstoffen gemäss Anspruch 1, dadurch gekennzeichnet, dass man in an sich bekannter Weise

a) eine Verbindung der Formel

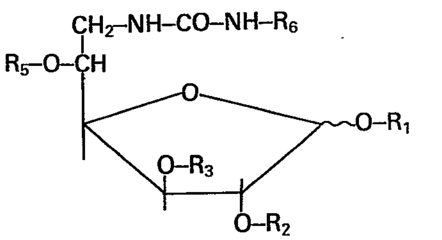

(II);

nitrosiert, oder

b) ein Amin der Formel

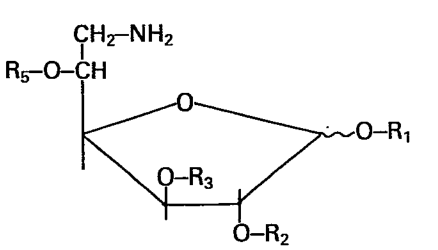

(III)

mit einem reaktionsfähigen Derivat einer N-Nitroso-N-$R_6$-carbaminsäure (IV) kondensiert.

21. Verbindungen, erhältlich nach den Verfahren des Anspruchs 20.

**Claims**

1. $N_1$-Glucofuranosid-6-yl-$N_3$-nitroso-ureas of the formula I

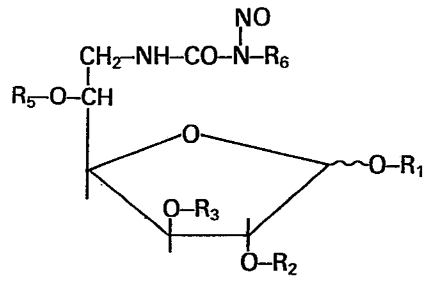

(I)

wherein $R_1$ and $R_2$ are each hydrogen, alkyl which is unsubstituted or substituted by free or etherified hydroxyl groups or halogen, or they are each aralkyl or acyl which are each unsubstituted or substituted by lower alkyl, free or etherified hydroxyl, halogen or trifluoromethyl, or $R_1$ and $R_2$ together are also alkylidene or cycloalkylidene, $R_3$ and $R_5$ are each alkyl which is unsubstituted or substituted by free or etherified hydroxyl groups or halogen, or they are each aralkyl or acyl which are each unsubstituted or substituted by lower alkyl, free or etherified hydroxyl, halogen or trifluoromethyl, or $R_3$ and $R_5$ together are also alkylidene or cycloalkylidene, and $R_6$ is alkyl which is unsubstituted or substituted by free or etherified hydroxyl groups or halogen.

2. Compounds according to Claim 1, wherein $R_1$ and $R_2$ are each hydrogen, lower alkyl which is unsubstituted or substituted by hydroxyl, lower alkoxy or halogen, or they are each benzyl which is unsubstituted or substituted by hydroxyl, lower alkoxy, halogen or trifluoromethyl, or $R_1$ and $R_2$ together are also lower alkylidene or cycloalkylidene having 5–6 carbon atoms, $R_3$ and $R_5$ are each lower alkyl which is unsubstituted or substituted by hydroxyl, lower alkoxy or halogen, or they are each benzyl which is unsubstituted or substituted by hydroxyl, lower alkoxy, halogen or trifluoromethyl, or they are each lower alkanoyl, or benzoyl which is unsubstituted or substituted by halogen, lower alkoxy, hydroxyl or lower alkanoyloxy, or together they are also lower alkylidene or cycloalkylidene having 5–6 carbon atoms, and $R_6$ is a lower alkyl group which is unsubstituted or substituted by halogen, hydroxyl or lower alkoxy.

3. Compounds according to Claim 2, wherein the substituted benzyl group is substituted in the para-position.

4. Compounds according to Claim 1, wherein $R_1$ is lower alkyl and $R_2$ is hydrogen, or $R_1$ and $R_2$ together are lower alkylidene, $R_3$ and $R_5$ are each lower alkyl, or benzyl which is unsubstituted or substituted by halogen, hydroxyl, lower alkoxy or alkyl, and $R_6$ is lower alkyl which is unsubstituted or substituted by chlorine.

5. Compounds according to Claim 1, wherein $R_1$ is hydrogen, methyl, ethyl or propyl, and $R_2$ is hydrogen, or $R_1$ and $R_2$ together are the isopropylidene group, and $R_3$ and $R_5$ are each methyl, and $R_6$ is methyl or chloroethyl.

6. The α- or β-anomers of the compounds stated in Claims 1–5.

7. Ethyl-3,5-di-O-methyl-6-deoxy-6-(3-methyl-3-nitrosoureido)-α-D-glucofuranoside or -β-D-glucofuranoside.

8. 6-Deoxy-3,5-di-O-methyl-1,2-O-isopropylidene-6-(3-methyl-3-nitroureido)-α-D-glucofuranose.

9. Ethyl-2-O-acetyl-6-deoxy-3,5-di-O-methyl-6-(3-methyl-3-nitrosoureido)-D-glucofuranoside.

10. Ethyl-6-(3-ethyl-3-nitrosoureido)-6-deoxy-3,5-di-O-methyl-D-glucofuranoside.

11. Ethyl-6-[3-(2-chloroethyl)-3-nitrosoureido]-6-deoxy-3,5-di-O-methyl-D-glucofuranoside.

12. Ethyl-6-(3-n-butyl-3-nitrosoureido)-6-deoxy-3,5-di-O-methyl-D-glucofuranoside.

13. Ethyl-6-deoxy-5-O-methyl-6-(3-methyl-3-nitrosoureido)-3-O-propyl-D-glucofuranoside.

14. Ethyl-5-O-ethyl-6-deoxy-6-(3-methyl-3-nitrosoureido)-3-O-propyl-D-glucofuranoside.

15. Ethyl-3-O-benzyl-6-deoxy-5-O-methyl-6-(3-methyl-3-nitrosoureido)-D-glucofuranoside.

16. Pharmaceutical preparations containing one of the compounds given in Claims 1 to 15.

17. Compounds according to Claim 1 for application in a process for the therapeutic treatment of the human or animal body.

18. Compounds according to Claim 1 as active substances inhibiting tumors.

19. The use of compounds according to Claim 1 for the production of pharmaceutical preparations.

20. A process for producing $N_1$-glucofuranosid-6-yl-$N_3$-nitroso-ureas according to Claim 1, which process comprises

a) introducing the nitroso group, in a manner known per se, into a compound of the formula

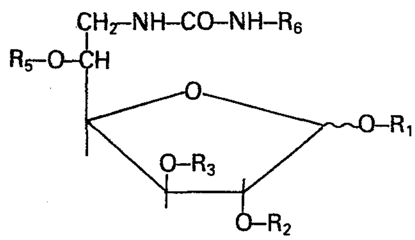

(II); or

b) condensing, in a manner known per se, an amine of the formula

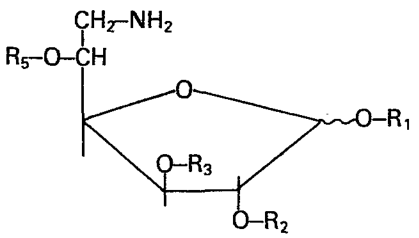

(III)

with a reactive derivative of an N-nitroso-N-$R_6$-carbamic acid (IV).

21. The compounds obtainable by the process according to Claim 20.

**Revendications**

1. $N_1$-glucofurannoside-6-yl-$N_3$-nitroso-urées de formule I:

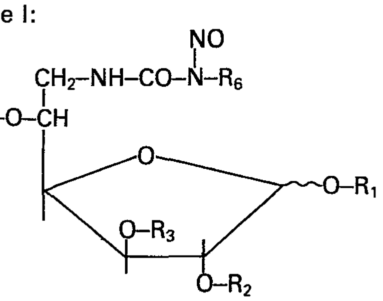

(I)

dans laquelle $R_1$ et $R_2$ représentent l'hydrogène, un groupe alkyle éventuellement substitué par des groupes hydroxy libres ou éthérifiés ou par des halogènes ou un groupe aralkyle ou acyle éventuellement substitué par des groupes alkyle inférieurs, hydroxy libre ou éthérifié, des halogènes ou des groupes trifluorométhyle, $R_1$ et $R_2$ représentent également ensemble un groupe alkylidène ou cycloalkylidène, $R_3$ et $R_5$ représentent un groupe alkyle éventuellement substitué par des groupes hydroxy libres ou éthérifiés ou par des halogènes ou un groupe aralkyle ou acyle éventuellement substitué par des groupes alkyles inférieurs, hydroxy libre ou éthérifié, des halogènes ou des groupes trifluorométhyle, $R_3$ et $R_5$ représentent également ensemble un groupe alkylidène ou cycloalkylidène, et $R_6$ représente un groupe alkyle éventuellement substitué par des groupes hydroxy libres ou éthérifiés ou par des halogènes.

2. Composés selon la revendication 1, dans lesquels $R_1$ et $R_2$ représentent l'hydrogène, un groupe alkyle inférieur éventuellement substitué par des groupes hydroxy, alcoxy inférieur ou des halogènes, un groupe benzyle éventuellement substitué par des groupes hydroxy, alcoxy inférieur, des halogènes ou des groupes trifluorométhyle, $R_1$ et $R_2$ représentent également un groupe alkylidène inférieur ou cycloalkylidène en C5 – C6, $R_3$ et $R_5$ représentent un groupe alkyle inférieur éventuellement substitué par des groupes hydroxy, alcoxy inférieur ou des halogènes, un groupe benzyle éventuellement substitué par des groupes hydroxy, alcoxy inférieur, des halogènes, ou des groupes trifluorométhyle, un groupe alcanoyle inférieur ou un groupe benzoyle éventuellement substitué par des halogènes, des groupes alcoxy inférieur, hydroxy ou alcanoyloxy inférieur, ou représentent ensemble un groupe alkylidène inférieur ou cycloalkylidène en C 5 – C 6, et $R_6$ représente un groupe alkyle inférieur éventuellement substitué par des halogènes, des groupes hydroxy ou alcoxy inférieur.

3. Composés selon la revendication 2, dans lesquels le groupe benzyle substitué est substitué en position para.

4. Composés selon la revendication 1, dans lesquels $R_1$ représente un groupe alkyle inférieur et $R_2$ l'hydrogène ou bien $R_1$ et $R_2$ représentent ensemble un groupe alkylidène inférieur, $R_3$ et $R_5$ représentent un groupe alkyle inférieur ou un

groupe benzyle éventuellement substitué par des halogènes, des groupes hydroxy, alcoxy inférieur ou alkyle et $R_6$ représente un groupe alkyle inférieur éventuellement substitué par le chlore.

5. Composés selon la revendication 1, dans lesquels $R_1$ représente l'hydrogène, un groupe méthyle, éthyle ou propyle et $R_2$ représente l'hydrogène, et $R_1$ et $R_2$ représentent ensemble le reste isopropylidène et $R_3$ et $R_5$ représentent un groupe méthyle et $R_6$ un groupe méthyle ou chloréthyle.

6. Les anomères alpha ou bêta des composés mentionnés dans les revendications 1 à 5.

7. L'éthyl-3,5-di-O-méthyl-6-désoxy-6-(3-méthyl-3-nitrosouréido)-alpha-D-glucofurannoside ou -bêta-D-glucofurannoside.

8. Le 6-désoxy-3,5-di-O-méthyl-1,2-O-isopropylidène-6-(3-méthyl-3-nitroso-uréido)-alpha-D-glucofurannose.

9. L'éthyl-2-O-acétyl-6-désoxy-3,5-di-O-méthyl-6-(3-méthyl-3-nitrosouréido)-D-glucofurannoside.

10. L'éthyl-6-(3-éthyl-3-nitrosouréido)-6-désoxy-3,5-di-O-méthyl-D-glucofurannoside.

11. L'éthyl-6-[3-(2-chloréthyl-3-nitrosouréido)]-6-désoxy-3,5-di-O-méthyl-D-glucofurannoside.

12. L'éthyl-6-(3-n-butyl-3-nitrosouréido)-6-désoxy-3,5-di-O-méthyl-D-glucofurannoside.

13. L'éthyl-6-désoxy-5-O-méthyl-6-(3-méthyl-3-nitrosouréido)-3-O-propyl-D-glucofurannoside.

14. L'éthyl-5-O-éthyl-6-désoxy-6-(3-méthyl-3-nitrosouréido)-3-O-propyl-D-glucofurannoside.

15. L'éthyl-3-O-benzyl-6-désoxy-5-O-méthyl-6-(3-méthyl-3-nitrosouréido)-D-glucofurannoside.

16. Compositions pharmaceutiques contenant l'un des composés mentionnés dans les revendications 1 à 15.

17. Composés selon la revendication 1, pour l'utilisation dans un procédé pour le traitement thérapeutique de l'organisme humain ou animal.

18. Composés selon la revendication 1, en tant qu'agents inhibiteurs des tumeurs.

19. Utilisation des composés selon la revendication 1 pour la préparation de compositions pharmaceutiques.

20. Procédé de préparation des $N_1$-glucofurannoside-6-yl-$N_3$-nitrosourées selon la revendication 1, caractérisé en ce que, de manière connue en soi:

a) on nitrose un composé de formule:

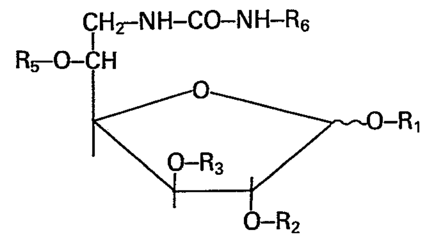

(II);

ou bien

b) on condense une amine de formule:

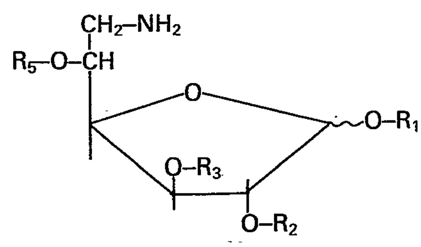

(III)

avec un dérivé réactif d'un

acide N-nitroso-N-$R_6$-carbamique (IV)

21. Composés susceptibles d'être obtenus par le procédé de la revendication 20.